# EUROPEAN PATENT APPLICATION

(11) **EP 1 553 095 A1**
(43) Date of publication of application: **13.07.2005**
(21) Application number: 03771273.4
(22) Date of filing: 17.07.2003
(51) Int. Cl.: C07D 319/20, C07C 303/28, C07C 309/66, C07C 41/03, C07C 41/16, C07C 43/23

(54) **PROCESS FOR INDUSTRIALLY PRODUCING OPTICALLY ACTIVE 1,4-BENZODIOXANE DERIVATIVE**

(30) Priority: 29.07.2002 JP 2002220152
(71) Applicant: KANEKA CORPORATION, Osaka-shi, Osaka 530-8288 (JP)
(72) Inventor: TANAKA, Tatsuyoshi, Takasago-shi, Hyogo 676-8688 (JP); MITSUDA, Masaru, Takasago-shi, Hyogo 676-8688 (JP)
(74) Representative: HOFFMANN - EITLE
(86) International application number: PCT/JP2003/009125
(87) International publication number: WO 2004/011451

(57) **Abstract**

A simple and safe method for producing optically active 1,4-benzodioxane derivatives useful as intermediates for pharmaceuticals and the like from inexpensive materials is provided.

An optically active triol compound (5) produced by reaction of catechol (2) and optically active 3-halogeno-1,2-propanediol (3) is sulfonylated to form optically active trisulfonate (6), followed by cyclization with a base to yield optically active 1,4-benzodioxane (1).

## Description

### Technical Field

The present invention relates to a method for producing optically active 1,4-benzodioxane derivatives useful as intermediates for pharmaceuticals, such as α-adrenergic antagonists and dopamine agonists.

### Background Art

The majority of conventional processes for producing optically active 1,4-benzodioxane derivatives, which are target compounds of the present invention, has a multi-step reaction and are very complex.

On the other hand, examples of relatively simple production processes include the following:
process (1) for resolving racemic 2-hydroxymethyl-2,3-dihydro-1,4-benzodioxane with lipase (Tetrahedron Lett. 33, 6283-6286 (1992));
process (2) for allowing catechol to react with optically active glycidyl tosylate in the presence of potassium carbonate or sodium hydride (Tetrahedron Letters, 29, 3671 (1988); Journal of Medicinal Chemistry, 32, 1402-1407 (1989); and EP 9402904 (corresponding to Japanese Unexamined Patent Application Publication No. 9-502431); a process for allowing catechol to react with optically active glycidyl nosylate in the presence of potassium carbonate (Japanese Unexamined Patent Application Publication No. 10-45746);
process (4) for allowing catechol to react with optically active glycidyl nosylate in the presence of a fluoride salt or in the presence of a catalytic amount of a fluoride salt and a theoretical amount of an inorganic alkali salt (Japanese Unexamined Patent Application Publication No. 2001-316385); and
process (5) for producing a 1,4-benzodioxane derivative by allowing monoalkylcatechol to react with optically active 3-chloro-1,3-propanediol to yield a diol derivative and then converting the resulting diol derivative into a sulfonated derivative or a cyclic carbonate derivative, followed by deprotection and cyclization (Japanese Unexamined Patent Application Publication No. 2001-81086, International Publication Nos. WO9851680 and WO9630360).

### Disclosure of Invention

Process (1) employs an optical resolution technique, thus leading to a low yield and low enantiomeric excess of the resulting compound.

Process (2) also has a low yield, and racemization proceeds.

In process (3), expensive glycidyl nosylate is used, and yield is low in crystallizing and purifying steps.

Process (4) provides a target product in high yield at high selectivity. However, expensive glycidyl nosylate and cesium fluoride must be used. In addition, the treatment of a waste solution containing fluorine is a problem.

In process (5), a deprotection procedure is performed by hydrogenation reaction, and an expensive palladium catalyst must be used. Furthermore, since this hydrogenation reaction requires a high-pressure hydrogen gas, which is explosive and flammable, there are problems with respect to facility and safety.

As described above, these processes have many problems in industrial production. Hence, the development of a better process has been desired.

In view of such present circumstances, it is an object of the present invention to produce an optically active 1,4-benzodioxane derivative, which is represented by general formula (1) described below, by a safe, efficient, and industrially advantageous method, from readily available materials.

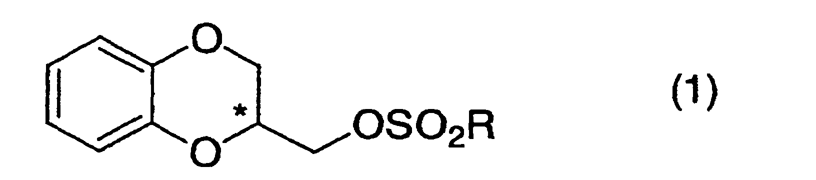

The present invention provides a method for producing an optically active 1,4-benzodioxane derivative represented by general formula (1): 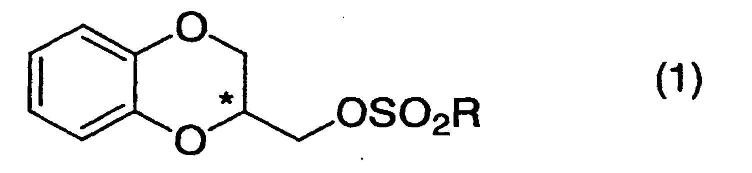 (where * represents an asymmetric center), the method including a first step of allowing catechol represented by formula (2): 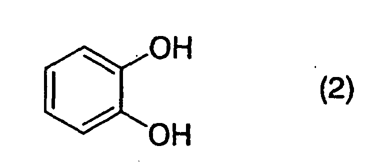 to react with an optically active 3-halogeno-1,2-propanediol represented by general formula (3): 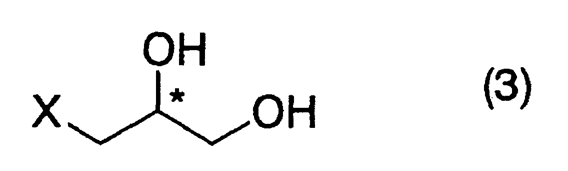 (where X represents a halogen atom; and * is the same as above), or an optically active glycidol represented by formula (4): 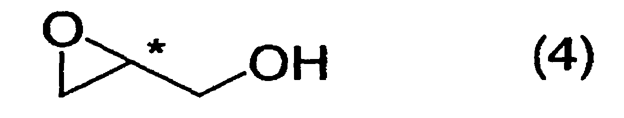 (where * is the same as above), in a solvent in the presence of a base, to yield an optically active triol compound represented by formula (5): 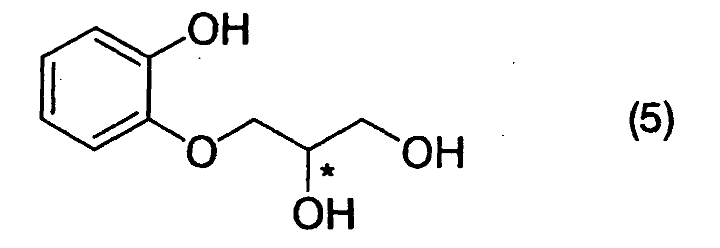 (where * is the same as above);
a second step of allowing the resulting compound to react with a sulfonylating agent in the presence of a tertiary amine to form an optically active trisulfonate compound represented by general formula (6): 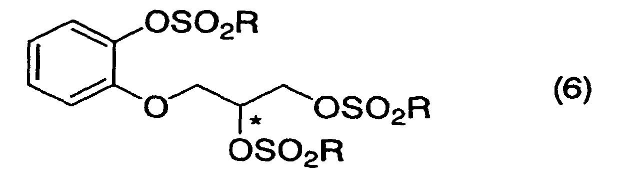 (where R represents an alkyl group having 1 to 12 carbon atoms or a phenyl group unsubstituted or substituted with a group having 1 to 12 carbon atoms; and * is the same as above); and
a third step of treating the resulting optically active trisulfonate compound with a base in a protic solvent or a mixed solvent of a protic solvent and an aprotic solvent to cause cyclization.

The present invention also provides a method for producing an optically active triol compound represented by formula (5): 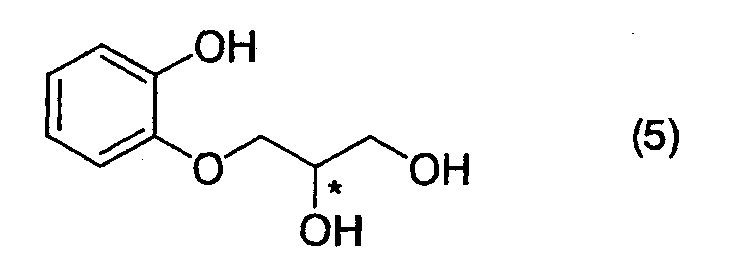 (where * represents an asymmetric center), the method including a step of allowing catechol represented by formula (2): 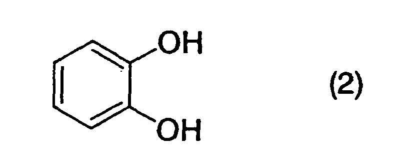 to react with an optically active 3-halogeno-1,2-propanediol represented by general formula (3): 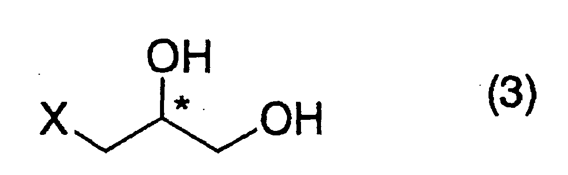 (where X represents a halogen atom; and * is the same as above), or to react with an optically active glycidol represented by formula (4): 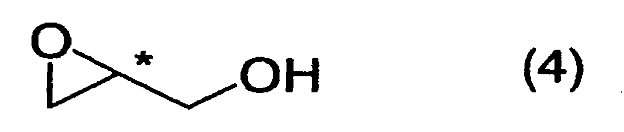 (where * is the same as above), in a solvent in the presence of a base.

The present invention provides a method for producing an optically active trisulfonate compound represented by general formula (6): 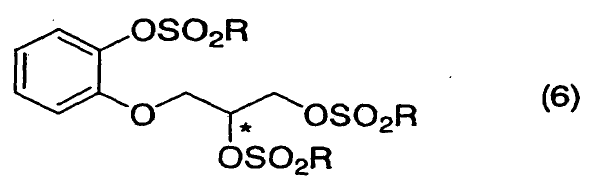 (where R represents an alkyl group having 1 to 12 carbon atoms or a phenyl group unsubstituted or substituted with a group having 1 to 12 carbon atoms; and * is the same as above), the method including a step of allowing an optically active triol compound represented by general formula (5): 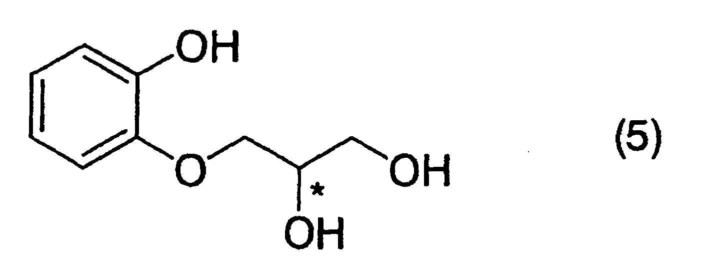 to react with a sulfonylating agent in the presence of a tertiary amine.

The present invention provides a method for producing an optically active 1,4-benzodioxane derivative represented by formula (1): 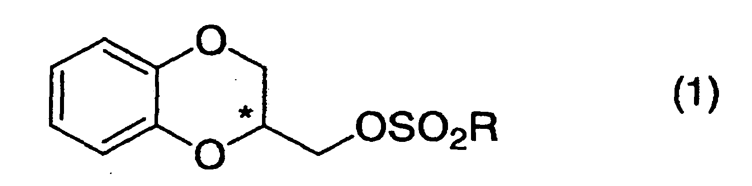 (where * represents an asymmetric center), the method including a step of treating an optically active trisulfonate compound represented by general formula (6): 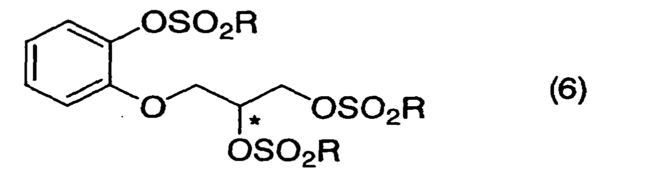 (where * is the same as above), with a base in a protic solvent or a mixed solvent of a protic solvent and an aprotic solvent to cause cyclization.

The present invention provides an optically active trisulfonate derivative represented by general formula (6): 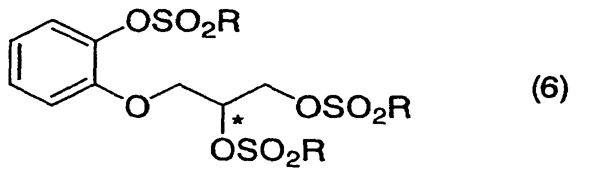 (where R represents an alkyl group having 1 to 12 carbon atoms or a phenyl group unsubstituted or substituted with a group having 1 to 12 carbon atoms).

### Best Mode for Carrying Out the Invention

The present invention will be described in detail below.

The scheme of a production method according to the present invention is shown below. 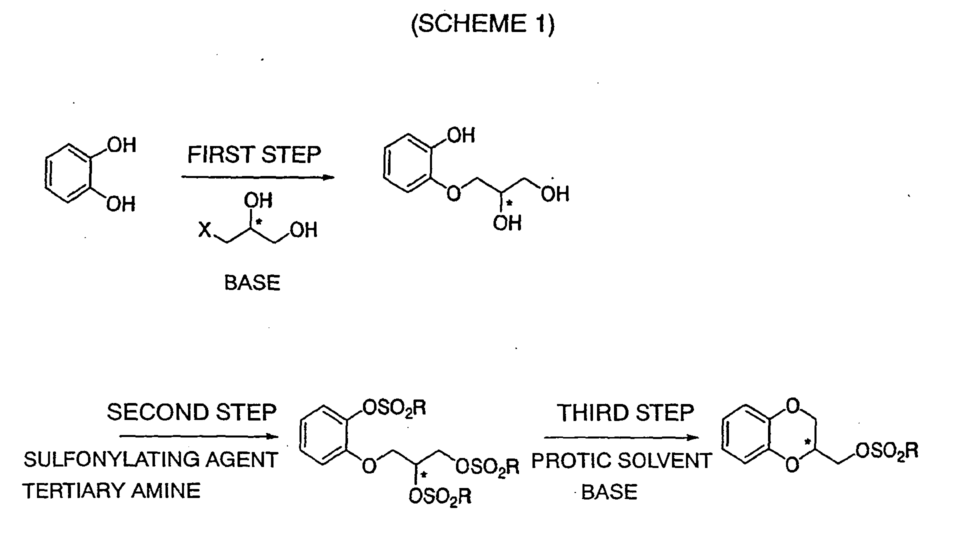 (where, in this scheme, X represents a halogen atom; R represents an alkyl group having 1 to 12 carbon atoms or a phenyl group unsubstituted or substituted with a group having 1 to 12 carbon atoms; and * represents an asymmetric center).

The first to third steps will be described in detail below.

### 1. First step

In this step, catechol (2): 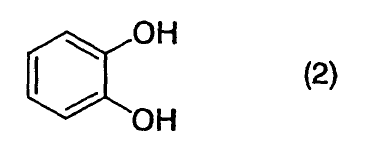 is allowed to react with optically active 3-halogeno-1,2-propanediol (3): 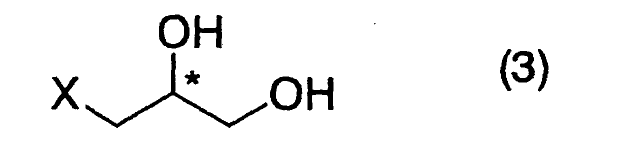 or optically active glycidol (4): 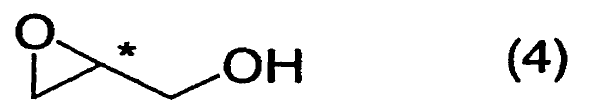 in a solvent in the presence of a base to yield an optically active triol compound (5):

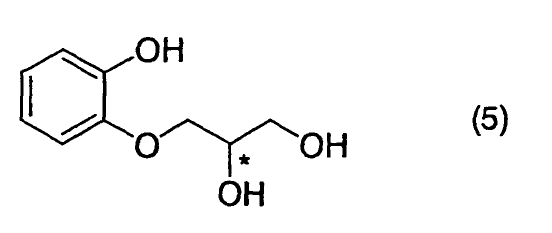

In optically active 3-halogeno-1,2-propanediol (3), X represents a halogen atom, for example, a chlorine atom, a bromine atom, or an iodine atom. In view of the ease of availability of materials, a chlorine atom and a bromine atom are preferable. A chlorine atom is more preferable.

Catechol (2) is used in an amount of 1 to 10 molar equivalents, preferably 2 to 3 molar equivalents, based on the amount of optically active 3-halogeno-1,2-propanediol (3) or optically active glycidol (4). When the amount of catechol (1) used is too low, self-polymerization of glycidol proceeds, thus decreasing the yield.

Since optically active glycidol (4) is unstable compared with optically active 3-halogeno-1,3-propanediol (3), in view of the yield and handling of these compounds, optically active 3-halogeno-1,3-propanediol (3) is preferably used.

Examples of the base used include, but are not limited to, metal amide compounds, such as lithium amide, sodium amide, lithium diisopropylamide, chloromagnesium isopropylamide, bromomagnesium isopropylamide, and chloromagnesium dicyclohexylamide; alkali metal compounds, such as methyllithium, *n*-butyllithium, methylmagnesium bromide, *i*-propylmagnesium chloride, and *tert*-butylmagnesium chloride; metal hydrides such as sodium hydride, potassium hydride, and calcium hydride; metal alkoxides such as sodium methoxide, sodium ethoxide, magnesium ethoxide, and potassium *tert*-butoxide; metal hydroxides, such as sodium hydroxide, potassium hydroxide, lithium hydroxide, magnesium hydroxide, and calcium hydroxide; and carbonate salts; such as sodium hydrogencarbonate, sodium carbonate, potassium carbonate, calcium carbonate, and magnesium carbonate. Among them, sodium hydride; metal hydroxides such as sodium hydroxide, potassium hydroxide, and lithium hydroxide; and metal alkoxides, such as sodium methoxide, sodium ethoxide, magnesium ethoxide, and potassium tert-butoxide, are inexpensive and preferable as the base used in this step.

The base is used in an amount of 1 to 10 molar equivalents, preferably 3 to 5 molar equivalents, based on catechol (2).

A solvent used in this step is not limited but is preferably an aprotic organic solvent when a metal amide, an alkali metal, or an alkali metal hydride is used as a base. When a metal alkoxide, a metal hydride, or a carbonate salt is used, either aprotic or protic solvent may be used.

Examples of the aprotic organic solvent include aprotic polar solvents, such as *N,N*-dimethylformamide (DMF), dimethyl sulfoxide, and hexamethylphosphoric triamide; ether solvents, such as diethyl ether, tetrahydrofuran (THF), 1,4-dioxane, methyl *tert*-butyl ether, dimethoxyethane, ethylene glycol, and dimethyl ether; aromatic hydrocarbon solvents, such as benzene, toluene, and xylene; hydrocarbon solvents such as *n*-pentane and *n-*hexane; nitrile solvents such as acetonitrile and butyronitrile; ester solvents such as ethyl acetate and butyl acetate; and ketone solvents such as acetone. Examples of the protic solvent include alcoholic solvents such as methanol, ethanol, isopropanol, and butanol; and water. These may be used alone or in combination.

In this step, particularly, sodium hydroxide is preferably used as a base, and methanol and water are preferably used as protic solvents.

The reaction temperature is set at 0°C to 100°C, preferably 20°C to 40°C. When the reaction temperature is too low, the rate of reaction is markedly reduced, which is inefficient. Excessively high reaction temperatures result in generation of by-products and are thus not preferable.

After the reaction, to separate the resulting product from the reaction mixture, standard workup may be performed. For example, a base is neutralized with a common inorganic salt, such as hydrochloric acid or sulfuric acid, and then an extracting operation is performed with a general extracting solvent, such as ethyl acetate, diethyl ether, methylene chloride, toluene, or hexane. The reaction solvent and the extracting solvent are removed from the resulting extracted solution by, for example, heating under reduced pressure to isolate a target compound. Alternatively, after the reaction, a reaction solvent is removed by, for example, heating under reduced pressure, and then the same operation may be performed. The target compound thus produced is substantially pure but may be further purified by a common technique, for example, crystallization, fractional distillation, or column chromatography, to achieve higher purity.

### 2. Second step

In this step, an optically active triol compound (5): 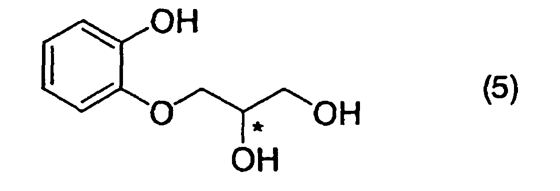 is subjected to sulfonylation of a hydroxyl group with a sulfonylating agent in an organic solvent in the presence of a tertiary amine to yield an optically active trisulfonate compound (6):

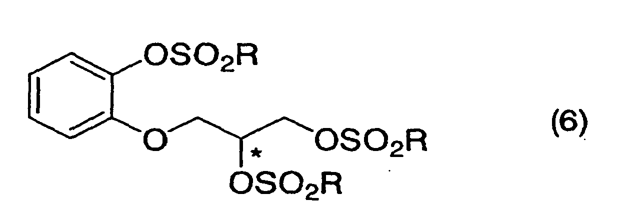

A known technique (for example, described in "*Protective Groups in Organic Synthesis"*, 2nd edition, Green, John Wiley & Sons, Inc.) may be applicable to a process for sulfonylation of a hydroxyl group.

Examples of the sulfonylating agent include sulfonyl halide compounds, such as benzenesulfonyl chloride, *p*-toluenesulfonyl chloride, *m*-nitrobenzenesulfonyl chloride, trifluoromethanesulfonyl chloride, and alkylsulfonyl chlorides each containing an alkyl group having 1 to 12 carbon atoms, such as methanesulfonyl chloride and dodecanesulfonyl chloride; and acid anhydrides, such as benzenesulfonic acid anhydride, *p*-toluenesulfonic acid anhydride, trifluoromethanesulfonic acid anhydride, and methanesulfonic acid anhydride.

The sulfonylating agent is used in an amount of 3 to 10 molar equivalents, preferably 3 to 5 molar equivalents, based on the amount of optically active triol compound (5).

Examples of the tertiary amine include trialkylamines containing 1 to 12 carbon atoms, for example, trimethylamine, triethylamine, and ethyldiisopropylamine; tertiary amines containing an alkyl group having 1 to 4 carbon atoms and a phenyl group, for example, *N,N-*dimethylaniline, *N,N*-diethylaniline, and *N.N*-dimethylaminopyridine; nitrogen-containing organic bases, such as pyridine, picoline, and lutidine; and *N,N,N,N*-tetramethyl-α,ω-alkyldiamines containing 1 to 10 carbon atoms, for example, *N,N,N,N*-tetramethyl-1,2-ethylenediamine, *N,N,N,N*-tetramethyl-1,3-propanediamine, and *N,N,N,N*-tetramethyl-1,6-hexanediamine, which may be used alone or in combination. The use of a mixed base containing triethylamine and *N,N,N,N*-tetramethyl-1,6-hexanediamine leads to high yield, thus being particularly preferable.

The amine is used in an amount of 0.1 to 10 molar equivalents, preferably 0.1 to 5 molar equivalents, based on the amount of optically active triol compound (5).

The reaction temperature is -20°C to 150°C, preferably 0° C to 50°C.

Examples of the organic solvent used include ether solvents, such as diethyl ether, tetrahydrofuran (THF), 1,4-dioxane, methyl *tert*-butyl ether, dimethoxyethane, ethylene glycol, and dimethyl ether; aromatic hydrocarbon solvents, such as benzene, toluene, and xylenes; hydrocarbon solvents, such as *n*-pentane and *n*-hexane; nitrile solvents, such as acetonitrile and butyronitrile; ester solvents, such as ethyl acetate and butyl acetate; halogenated solvents, such as dichloromethane, 1,2-dichloroethane, 1,1,1-trichloroethane, and chloroform; aprotic polar solvents, such as dimethylformamide, *N*-methylpyrrolidone, and hexamethylphosphoric triamide; alcoholic solvents, such as methanol, ethanol, isopropanol, and *n*-butanol; and water, which may be used alone or in combination. Among them, acetonitrile is particularly preferable.

After the reaction, to separate the resulting product from the reaction mixture, standard workup may be performed. For example, water is added to the resulting mixture after the reaction, and then an extracting operation is performed with a general extracting solvent, such as ethyl acetate, diethyl ether, methylene chloride, toluene, or hexane. The reaction solvent and the extracting solvent are removed from the resulting extracted solution by, for example, heating under reduced pressure to isolate a target compound. Alternatively, after the reaction, the reaction solvent is removed by, for example, heating under reduced pressure, and then the same operation may be performed. The target compound thus produced is substantially pure but may be further purified by a common technique, for example, crystallization, fractional distillation, or column chromatography, to achieve higher purity.

### 3. Third step

In this step, the optically active trisulfonate compound (6): 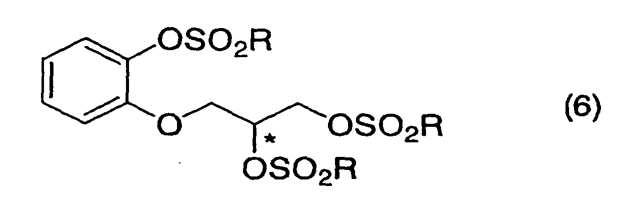 is treated with a base in a protic solvent or a mixed solvent containing a protic solvent and an aprotic solvent to yield an optically active 1,4-benzodioxane derivative (1):

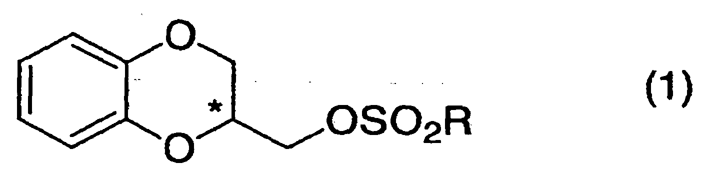

Examples of the base used include, but are not limited to, metal hydroxides such as sodium hydroxide, potassium hydroxide, lithium hydroxide, magnesium hydroxide, and barium hydroxide; carbonates, such as sodium carbonate, potassium carbonate, and sodium hydrogencarbonate; metal amide compounds, such as lithium amide, sodium amide, lithium diisopropylamide, chloromagnesium diisopropylamide, bromomagnesium isopropylamide, and chloromagnesium dicyclohexylamide; alkali metal compounds, such as methyllithium, *n*-butyllithium, methylmagnesium bromide, *i*-propylmagnesium chloride, and *tert*-butylmagnesium chloride; metal alkoxides such as sodium methoxide, sodium ethoxide, magnesium ethoxide, and potassium *tert*-butoxide; metal hydrides such as lithium hydride, sodium hydride, potassium hydride, and calcium hydride; and amines, such as triethylamine, diisopropylethylamine, *N*-methylmorpholine, dimethylaniline, and pyridine. Sodium hydride; metal hydroxides, such as sodium hydroxide, potassium hydroxide, and lithium hydroxide; and metal alkoxides, such as sodium methoxide, sodium ethoxide, magnesium ethoxide, and potassium *tert-*butoxide, are inexpensive and preferable as the base used in this step.

The base is used in an amount of 2 to 30 molar equivalents, preferably 3 to 12 molar equivalents, based on the optically active trisulfonate compound (6).

The reaction temperature is 0°C to 100°C, preferably 20° C to 40° C.

A protic solvent or a mixed solvent containing a protic solvent can be used as a reaction solvent in this step. Examples of the protic solvent include water; and alcoholic solvents such as methanol, ethanol, isopropanol, and *n*-butanol. Water and methanol are inexpensive and each preferable as a solvent used in this step. Alternatively, the mixed solvent may further contain an aprotic solvent. Examples of the aprotic solvent include hydrocarbon solvents, such as benzene, toluene, *n*-hexane, and cyclohexane; ether solvents, such as diethyl ether, tetrahydrofuran (THF), 1,4-dioxane, methyl *tert*-butyl ether, dimethoxyethane, ethylene glycol, and dimethyl ether; halogenated solvents, such as methylene chloride, chloroform, 1,1,1-trichloroethane, and 1,2-dichloroethane; and aprotic polar solvents, such as dimethylformamide, *N*-methylpyrrolidone, and hexamethylphosphoric triamide. These may be used alone or in combination.

After the reaction, to separate the resulting product from the reaction mixture, standard workup may be performed. For example, water is added to the resulting mixture after the reaction, and then an extracting operation is performed with a general extracting solvent, such as ethyl acetate, diethyl ether, methylene chloride, toluene, or hexane. The reaction solvent and the extracting solvent are removed from the resulting extracted solution by, for example, heating under reduced pressure to isolate a target compound. Alternatively, after the reaction, the reaction solvent is removed by, for example, heating under reduced pressure, and then the same operation may be performed. The target compound thus produced is substantially pure but may be further purified by a common technique, for example, crystallization, fractional distillation, or column chromatography, to achieve higher purity.

Hereinbefore, each step in the production method of the present invention was described in detail.

The optically active trisulfonate compound (6): 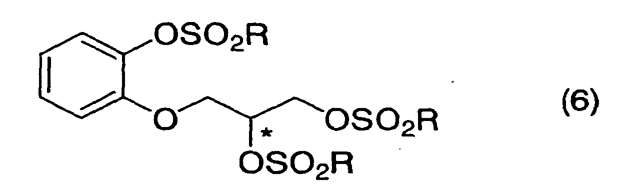 is a novel compound which is not described in any literature. The optically active trisulfonate compound can be readily induced by subjecting hydroxyl groups in the optically active triol compound (5): 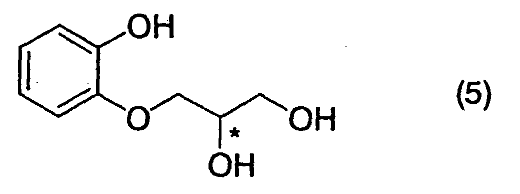 , which can be efficiently produced in the first step of the present invention, to sulfonylation according to a known process (for example, described in "*Protective Groups in Organic Synthesis"*, 2nd edition, Green, John Wiley & Sons, Inc.). As a result of the studies by the present inventors, the optically active trisulfonate compound (6) was the first compound produced by the above-described production method, and use as a pharmaceutical intermediate was developed.

R represents an alkyl group having 1 to 12 carbon atoms; or a phenyl group unsubstituted or substituted with a group having 1 to 12 carbon atoms. Examples of R include a methyl group, a phenyl group, a *p*-tolyl group, a nitrophenyl group, a methoxyphenyl group, and a trifluoromethanemethyl group. A *p*-tolyl group is preferable. * represents an asymmetric center. When such an optically active trisulfonate compound is used as an intermediate for medicine, such as α-adrenergic antagonist and dopamine agonist, the (R)-configuration is preferable with respect to the configuration of the asymmetric center.

The present invention will now be described in detail based on examples. The present invention is not limited to these examples.

### EXAMPLE 1 (R)-3-(2-hydroxyphenoxy)-1,2-propanediol

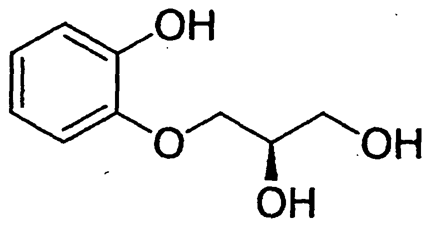

A 3 M aqueous solution of sodium hydroxide (75 ml, 226.1 mmol) was added dropwise to a solution of (R)-3-chloro-1,2-propanediol (5.0 g, 45.2 mmol)(98.2% e.e.) and catechol (10.0 g, 90.4 mmol) in water (25 ml) over a period of 3.5 hours at room temperature. After the dropwise addition, stirring was continued for 3 hours. Concentrated hydrochloric acid was added dropwise to the resulting reaction mixture at 0°C to adjust the pH in the system to 1.0. Subsequently, extraction was performed with ethyl acetate, and then the organic layer was washed with a saturated aqueous solution of sodium chloride, followed by drying over anhydrous sodium sulfate. The solvent was distilled off under reduced pressure, and then purification was performed by silica gel chromatography (hexane:ethyl acetate=1:1) to yield 6.7 g of a desired (R)-3-(2-hydroxyphenoxy)-1,2-propanediol (yield: 81%).
1H-NMR (400 MHz, DMSO-d6) δ3.79-3.84 (2H, m), 3.94-4.00 (2H, m), 4.66 (1H, dd, J=5.6, 5.6 Hz), 4.99 (1H, m), 6.69-6.90 (4H, m), 8.64 (1H, m).
13C-NMR (100 MHz, DMSO-d6) δ62.5, 69.9, 70.5, 113.3, 115.3, 119.1, 121.1, 146.6, 146.6.

### EXAMPLE 2 (R)-1,2-di(4-tolylsulfonyloxy)-3-[2-(4-tolylsulfonyloxy)phenoxy]propane

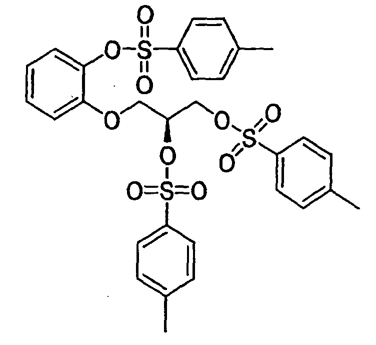

*p*-Toluenesulfonyl chloride (20.9 mg, 110.1 mmol) in the form of a solid was added to a solution of (R)-3-(2-hydroxyphenoxy)-1,2-propanediol (4.5 g, 24.4 mmol) produced in Example 1, triethylamine (11.1 g, 110.0 mmol), and *N,N,N,N*-tetramethylhexanediamine (1.26 g, 7.33 mmol) in acetonitrile (30 ml) at 0°C. After the addition, stirring was continued for 1 hour at 0°C and then for 2 hours at room temperature. Water (70 ml) was added to the resulting reaction mixture, followed by extraction with ethyl acetate. The organic layer was washed with saturated sodium chloride and then dried over anhydrous sodium sulfate. The solvent was distilled off under reduced pressure, and then purification was performed by silica gel chromatography (hexane:ethyl acetate=10:3) to yield 14.5 g of a desired (R)-1,2-di(4-tolylsulfonyloxy)-3-[2-(4-tolylsulfonyloxy)phenoxy]propane (yield: 92%).
1H-NMR (400 MHz, CDCl3) δ2.41 (3H, s), 2.45 (6H, s), 3.89-3.97 (2H, m), 4.11-4.20 (2H, m), 4.68 (1H, dt, J=7.4, 5.2 Hz), 6.74 (1H, dd, J=8.4, 1.2 Hz), 6.91 (1H, dt, J=1.6, 8.0 Hz), 7.06 (1H, dd, J=8.0, 1.6 Hz), 7.15 (1H, dt, J=8.0, 1.6 Hz), 7.26-7.34 (6H, m), 7.63-7.77 (6H, m).
13C-NMR (100 MHz, CDCl3) δ21.3, 21.7, 66.0, 67.1, 75.3, 113.9, 121.8, 124.0, 127.9, 128.3, 129.6, 130.0, 131.9, 132.5, 132.8, 138.4, 145.1, 145.3, 145.4, 149.8.

### EXAMPLE 3 (R)-2-[(4-methylphenylsulfonyloxy)methyl]-1,4-benzodioxane

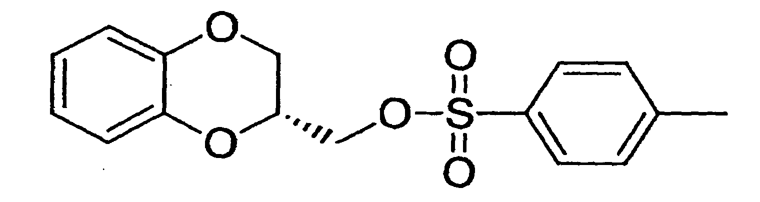

Sodium methoxide (202.4 mg, 3.7 mmol) in the form of a solid was added to a solution of (R)-1,2-di(4-tolylsulfonyloxy)-3-[2-(4-tolylsulfonyloxy)phenoxy]propane (807.4 mg, 1.25 mmol) produced in Example 2 in a mixed solvent (16 ml) of methanol and THF (5:3) at room temperature. After stirring was continued for 20 hours, sodium methoxide (607.0 mg, 11.2 mmol) was further added every 2 hours in three additions. Water was added to the resulting reaction mixture, followed by extraction with ethyl acetate. The organic layer was washed with saturated sodium chloride and then dried over anhydrous sodium sulfate. The solvent was distilled off under reduced pressure, and then purification was performed by silica gel chromatography (hexane:ethyl acetate=6:1) to yield 274.9 mg of a desired (R)-2-[(4-methylphenylsulfonyloxy)methyl]-1,4-benzodioxane (yield: 69%, 98.0% e.e.).
1H-NMR (400 MHz, CDCl3) δ2.45 (3H, s), 4.01-4.06 (1H, m), 4.17-4.26 (3H, m), 4.36-4.40 (1H, m), 6.77-6.90 (4H, m), 7.35 (2H, d, J=8.4 Hz), 7.80 (2H, d, J=8.4 Hz).
13C-NMR (100 MHz, CDCl3) δ21.7, 64.3, 67.1, 70.3, 117.2, 117.2, 121.6, 121.8, 127.9, 129.9, 132.3, 142.1, 142.6, 145.1.

### EXAMPLE 4 (R)-1,2-di(4-tolylsulfonyloxy)-3-[2-(4-tolylsulfonyloxy)phenoxy]propane

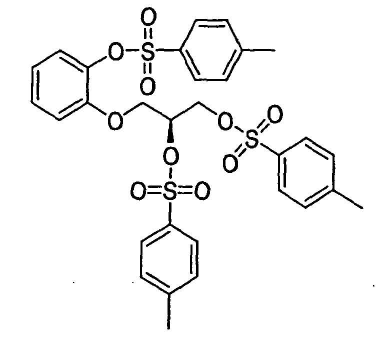

*p*-Toluenesulfonyl chloride (1025 mg, 537.5 mmol) in the form of a solid was added to a solution of (R)-3-(2-hydroxyphenoxy)-1,2-propanediol (300.5 mg, 1.63 mmol) produced in Example 1 in pyridine (1.0 ml) at 0°C. After the addition, stirring was continued for 1 hour at 0°C and then for 21 hours at room temperature. Water (70 ml) was added to the resulting reaction mixture, followed by extraction with ethyl acetate. The organic layer was washed with saturated sodium chloride and then dried over anhydrous sodium sulfate. The solvent was distilled off under reduced pressure, and then purification was performed by silica gel chromatography (hexane:ethyl acetate=3:1) to yield 512.4 mg of a desired (R)-1,2-di(4-tolylsulfonyloxy)-3-[2-(4-tolylsulfonyloxy)phenoxy]propane (yield: 49%).

### EXAMPLE 5 (R)-1,2-di(methylsulfonyloxy)-3-[2-(methylsulfonyloxy)phenoxy]propane

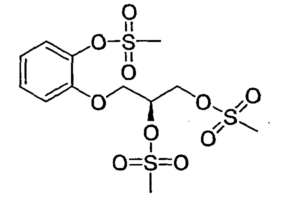

A solution of methanesulfonyl chloride (2.05 g, 17.92 mmol) in THF (3 ml) was added dropwise to a solution of (R)-3-(2-hydroxyphenoxy)-1,2-propanediol (1.0 g, 5.43 mmol) produced in Example 1 and triethylamine (2.47 g, 24.43 mmol) in THF (7 ml) over a period of 10 minutes at 0°C. After the addition, stirring was continued for 1 hour at 0°C, and then water (20 ml) was added to the resulting reaction solution, followed by extraction with ethyl acetate. The organic layer was washed with saturated sodium chloride and then dried over anhydrous sodium sulfate. The solvent was distilled off under reduced pressure, and then purification was performed by silica gel chromatography (hexane:ethyl acetate=10:3) to yield 2.05g of a desired (R)-1,2-di(methylsulfonyloxy)-3-[2-(methylsulfonyloxy)phenoxy]propane (yield: 90%).
1H-NMR (400 MHz, CDCl3) δ3.10 (3H, s), 3.18 (1H, s), 3.21 (1H, s), 4.25-4.37 (2H, m), 4.50-4.66 (2H, m), 5.18-5.23 (1H, m), 7.00-7.07 (2H, m), 7.25-7.31 (2H, m).
13C-NMR (100 MHz, CDCl3) δ31.7, 38.3, 38.7, 67.2, 67.7, 76.0, 113.0, 122.5, 124.0, 128.5, 138.3, 149.9.

### EXAMPLE 6 (R)-2-[(4-methylphenylsulfonyloxy)methyl]-1,4-benzodioxane

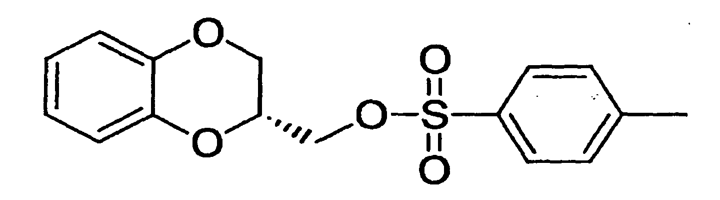

A 3 M aqueous solution of sodium hydroxide (2.0 ml, 6.0 mmol) was added dropwise to a solution of (R)-1,2-di(4-tolylsulfonyloxy)-3-[2-(4-tolylsulfonyloxy)phenoxy]propane (750.0 mg, 1.16 mmol) produced in Example 2 in a mixed solvent (18 ml) of methanol and THF (1:1) over a period of 5 minutes at room temperature. After stirring was continued for 26 hours at an external temperature of 40°C, water was added to the resulting reaction solution, followed by extraction with ethyl acetate. The organic layer was washed with saturated sodium chloride and then dried over anhydrous sodium sulfate. The solvent was distilled off under reduced pressure, and then purification was performed by silica gel chromatography (hexane:ethyl acetate=6:1) to yield 123.1 mg of a desired (R)-2-[(4-methylphenylsulfonyloxy)methyl]-1,4-benzodioxane (yield: 33%, 98.0% e.e.).

### Industrial Applicability

The present invention provides a safe method for producing an optically active 1,4-benzodioxane derivative from inexpensive materials with high efficiency. Furthermore, the optically active trisulfonate compound (6) is the first compound produced by the method, and use as a pharmaceutical intermediate was developed.

## Claims

1. A method for producing an optically active 1,4-benzodioxane derivative represented by general formula (1): 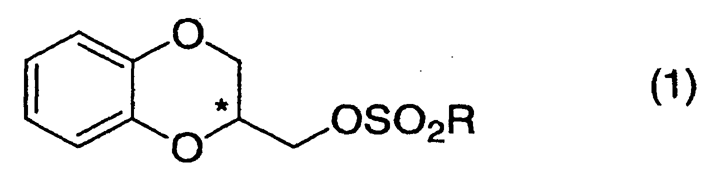 (where * represents an asymmetric center), the method comprising:
a first step of allowing catechol represented by formula (2): 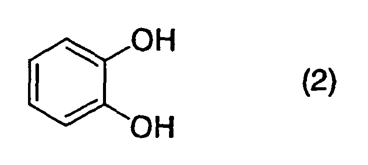 to react with an optically active 3-halogeno-1,2-propanediol represented by general formula (3): 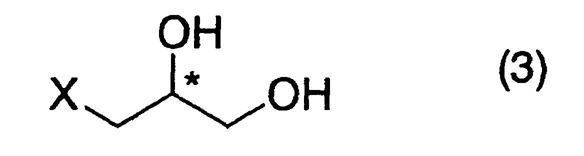 (where X represents halogen atom; and * is the same as above), or an optically active glycidol represented by formula (4): 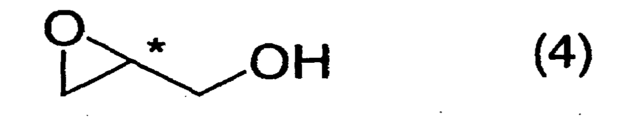 (where * is the same as above), in a solvent in the presence of a base, to yield an optically active triol compound represented by formula (5): 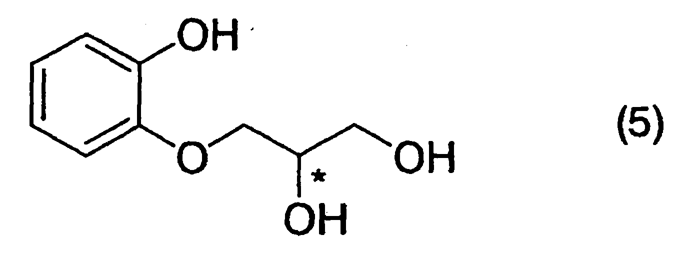 (where * is the same as above);
a second step of allowing the resulting compound to react with a sulfonylating agent in the presence of a tertiary amine to form an optically active trisulfonate compound represented by general formula (6): 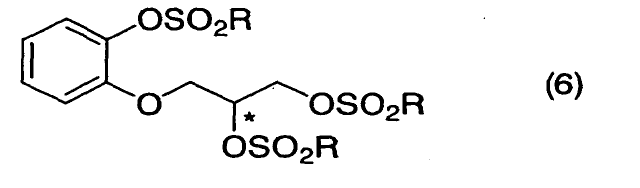 (where R represents an alkyl group having 1 to 12 carbon atoms or a phenyl group unsubstituted or substituted with a group having 1 to 12 carbon atoms; and * is the same as above); and
a third step of treating the resulting optically active trisulfonate compound with a base in a protic solvent or a mixed solvent of a protic solvent and an aprotic solvent to cause cyclization.

2. The method for producing an optically active 1,4-benzodioxane derivative according to Claim 1, wherein X represents a chlorine atom.

3. The method for producing an optically active 1,4-benzodioxane derivative according to Claim 1 and 2, wherein, in the first step, an alkali metal hydroxide is used as the base.

4. The method for producing an optically active 1,4-benzodioxane derivative according to Claims 1 to 3, wherein, in the first step, water is used as the solvent.

5. The method for producing an optically active 1,4-benzodioxane derivative according to any one of Claims 1 to 4, wherein, in the second step, the sulfonylating agent is arylsulfonyl chloride containing 6 to 12 carbon atoms or alkylsulfonyl chloride containing 1 to 12 carbon atoms.

6. The method for producing an optically active 1,4-benzodioxane derivative according to Claims 1 to 4, wherein, in the second step, the sulfonylating agent is *p*-toluenesulfonyl chloride.

7. The method for producing an optically active 1,4-benzodioxane derivative according to any one of Claims 1 to 6, wherein, in the second step, a mixed amine containing triethylamine and *N,N,N,N*-tetramethyl-1,6-hexanediamine is used as the tertiary amine.

8. The method for producing an optically active 1,4-benzodioxane derivative according to any one of Claims 1 to 7, wherein, in the third step, sodium alkoxide containing 1 to 4 carbon atoms is used as the base.

9. The method for producing an optically active 1,4-benzodioxane derivative according to Claim 8, wherein the sodium alkoxide is sodium methoxide.

10. The method for producing an optically active 1,4-benzodioxane derivative according to Claims 1 to 9, wherein, in the third step, a mixed solvent of an alcohol containing 1 to 4 carbon atoms and tetrahydrofuran is used as the mixed solvent of a protic solvent and an aprotic solvent.

11. The method for producing an optically active 1,4-benzodioxane derivative according to Claim 10, wherein the mixed solvent of a protic solvent and an aprotic solvent is a mixed solvent of methanol and tetrahydrofuran.

12. The method for producing an optically active 1,4-benzodioxane derivative according to Claims 1 to 11, wherein the optically active 3-halogeno-1,3-propanediol has (R) configuration.

13. A method for producing an optically active triol compound represented by formula (5): 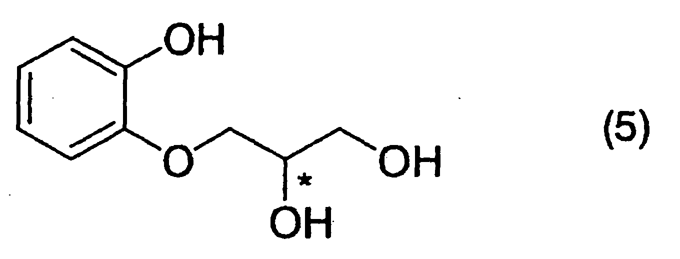 (where * represents an asymmetric center), the method comprising a step of:
allowing catechol represented by formula (2): 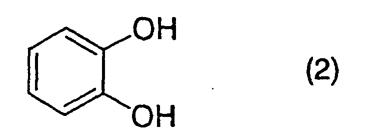 to react with an optically active 3-halogeno-1,2-propanediol represented by general formula (3): 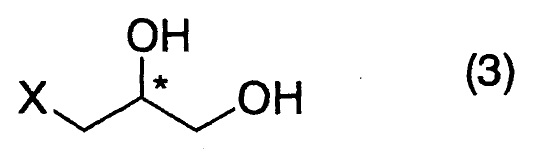 (where X represents a halogen atom; and * is the same as above), or an optically active glycidol represented by formula (4): 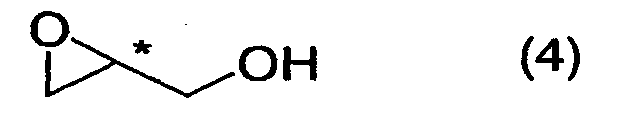 (where * is the same as above), in a solvent in the presence of a base.

14. The method according to Claim 13, wherein sodium hydroxide is used as the base.

15. The method according to Claim 13 and 14, wherein water is used as the solvent.

16. The method according to Claims 13 to 15, wherein X represents a chlorine atom.

17. A method for producing an optically active trisulfonate compound represented by general formula (6): 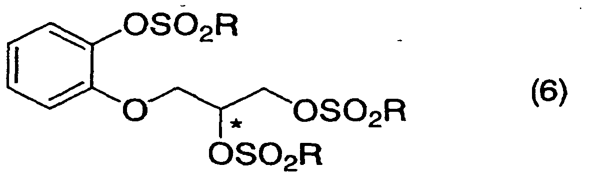 (where R represents an alkyl group having 1 to 12 carbon atoms or a phenyl group unsubstituted or substituted with a group having 1 to 12 carbon atoms; and * is the same as above), the method comprising a step of:
allowing an optically active triol compound represented by general formula (5): 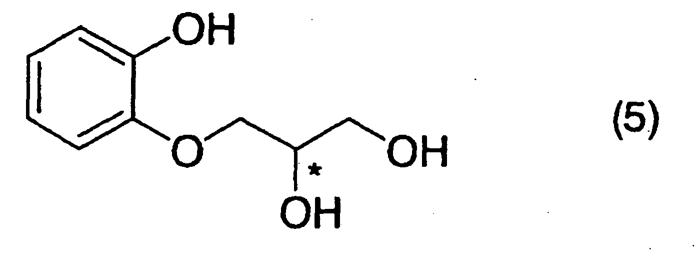 to react with a sulfonylating agent in the presence of a tertiary amine.

18. The method according to Claim 17, wherein the sulfonylating agent is arylsulfonyl chloride containing 6 to 12 carbon atoms or alkylsulfonyl chloride containing 1 to 12 carbon atoms.

19. The method according to Claim 18, wherein the sulfonylating agent is *p*-toluenesulfonyl chloride.

20. The method according to any one of Claims 17 to 19, wherein a mixed amine of triethylamine and *N,N,N,N*-tetramethyl-1,6-hexanediamine is used as the tertiary amine.

21. A method for producing an optically active 1,4-benzodioxahe derivative represented by formula (1): 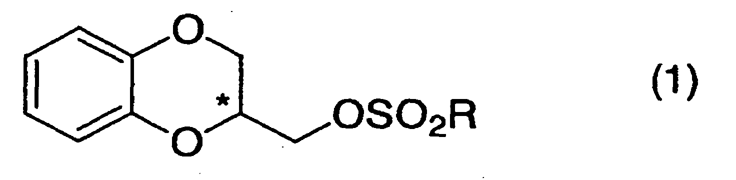 (where * represents an asymmetric center), the method comprising a step of:
treating an optically active trisulfonate compound represented by general formula (6): 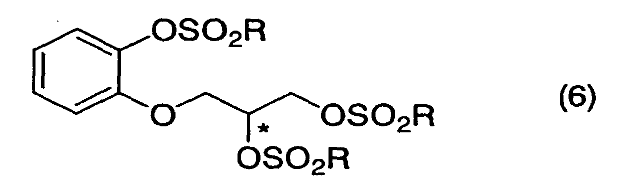 (where * is the same as above), with a base in a protic solvent or a mixed solvent of a protic solvent and an aprotic solvent to cause cyclization.

22. The method according to Claim 21, wherein sodium alkoxide containing 1 to 4 carbon atoms is used as the base.

23. The method according to Claim 21, wherein the base is sodium methoxide.

24. The method according to Claims 21 to 23, wherein a mixed solvent of an alcohol containing 1 to 4 carbon atoms and tetrahydrofuran is used as the mixed solvent of a protic solvent and an aprotic solvent.

25. The method according to Claims 21 to 23, wherein a mixed solvent of methanol and tetrahydrofuran is used as the mixed solvent of a protic solvent and an aprotic solvent.

26. An optically active trisulfonate derivative represented by general formula (6): 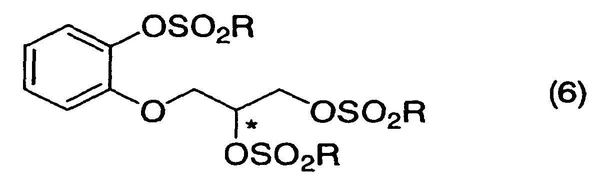 (where R represents an alkyl group having 1 to 12 carbon atoms or a phenyl group unsubstituted or substituted with a group having 1 to 12 carbon atoms).

27. The derivative according to Claim 26, wherein R represents *p*-tolyl.
